# EUROPEAN PATENT SPECIFICATION

(11) **EP 0 318 736 B1**
(45) Date of publication and mention of the grant of the patent: **28.07.1993**
(21) Application number: 88118725.6
(22) Date of filing: 10.11.1988
(51) Int. Cl.: C07C 2/26

(54) **Process for the oligomerization of internal olefins**
Verfahren zur Herstellung von internen Olefinen
Procédé d'oligomérisation d'oléfines internes

(30) Priority: 20.11.1987 IT 2270987
(43) Date of publication of application: 07.06.1989
(73) Proprietor: Enichem Anic S.r.l., 90139 Palermo (IT)
(72) Inventor: Messina, Giuseppe, Alghero (Sassari) (IT); Lorenzoni, Loreno, Porto Torres (Sassari) (IT); Virdis, Angelo, Usini (Sassari) (IT); Dessantis, Nino, Sassari (IT)
(74) Representative: Cioni, Carlo

(56) References cited:
- CHEMICAL ABSTRACTS, vol. 98, no. 5, January 1983, page 619, abstract no. 34211h, Columbus, Ohio, US; & JP-A-57 140 729
- CHEMICAL ABSTRACTS, vol. 101, no. 1, July 1984, page 562, abstract no. 6597a, Columbus, Ohio, US; & JP-A-59 21 629
- CHEMICAL ABSTRACTS, vol. 101, no. 1, July 1984, page 562, abstract no. 6598b, Columbus, Ohio, US; & JP-A-59 21 628
- CHEMICAL REVIEWS, 1977, vol. 77, no. 1, pages 69-92
- 3M TECHNICAL INFORMATION (Trifluoromethanesulfonic Acid)

## Description

The present invention refers to a process for the oligomerization of olefins using trifluoromethanesulphonic acid monohydrate as the reaction catalyst. Oligomers for high-viscosity lubricants are known to be prepared by cationic oligomerization of suitably selected α-olefins.

In particular, it is known that cationic oligomerization of decene-1 leads to an oligomer, trimer being the major product, which, upon hydrogenation, is a very good base for synthetic lubricants with excellent characteristics as far as viscosity index, viscosity at 210°F, volatility, pour point, etc., are concerned.

The remarkable rheological properties of such a product depend on a strict control of the molecular structure (branched chains of suitable lenght) as well as of the molecular weight and the molecular weight distribution. Low molecular weights would in fact increase product volatility, while the presence of higher oligomers would cause an increase in viscosity to unacceptable levels.

The α-olefin oligomers, however, owing to the high cost of the starting α-olefins, are very expensive. Therefore, products with similar rheological characteristics, but prepared from less expensive and more readily available monomers, are sought.

Oligomerization of internal olefins with acid catalysts stronger than those generally employed for the oligomerization of α-olefins is also known. The advantage of starting from internal olefins, which are commonly obtained from linear alkanes by widely known dehydrogenation processes, instead of α-olefins, resides in their low cost and ready availability. Oligomerization of internal olefins catalyzed by Friedel-Craft acids, typically AlCl₃, however leads to oligomers with a broad molecular weight distribution, unsuitable for use as lubricant oil bases, unless costly fractionation steps are applied to.

A new process for the oligomerization of olefins has recently been described (see Italian patent application 20106 A/80) which affords almost exclusively dimers, trimers and tetramers of the starting olefins, so that, starting from properly selected olefin fractions, typically within the C₁₀-C₁₅ range, oligomeric mixtures are obtained which can suitably be employed in the lubricant field. Said oligomers in fact show excellent rheological properties, remarkably better than those of the products obtained in the oligomerization catalyzed by conventional Friedel-Craft catalysts. Furthermore, the costly fractionation steps are avoided and almost complete conversion of the starting monomers is obtained.

The catalytic systems used in said process are AlCl₃ adducts with organic acid esters or BF₃ complexes with organic or inorganic acids.

According to the teachings there contained, particularly suitable to afford oligomers with a narrow molecular weight distribution, are BF₃ complexes with inorganic acids (e.g. BF₃·H₃PO₄).

Also these catalytic systems, however, suffer from some disadvantages.

First of all, the highly corrosive properties of the Al and B complexes create remarkable corrosion problems; secondly, it is necessary to use rigorously anhydrous monomer fractions (to avoid development of HCl or HF upon decomposition of the catalytic complex), and to carefully purify the obtained oligomers, removing any catalyst therefrom, mainly BF₃ traces, before the hydrogenation step (to avoid corrosion of the reactor and progressive poisoning of the hydrogenation catalyst); finally, the catalyst cannot be recycled because it cannot be recovered from the reaction mixture (these Al and B complexes in fact decompose before distilling).

A process for the oligomerization of olefins has been proposed in the Japanese application published JP 84/21,629 in which trifluoromethanesulphonic acid is used as catalyst. However the use of trifluoromethanesulphonic acid imposes reaction temperature ranging from -40 to 40°C, preferably from -20 to 10°C implying expensive refrigeration systems.

It has now surprisingly been found that using trifluoromethanesulphonic acid monohydrate as the reaction catalyst in the oligomerization of olefins, the above disadvantages can be overcome while, on the other hand, starting from suitably selected n-olefins, oligomers with rheological and structural properties similar to those of the oligomers obtained from α-olefins or from the same n-olefins but using AlCl₃ and BF₃ complexes, are obtained.

Trifluoromethanesulphonic acid monohydrate is in fact strong enough to oligomerize n-olefins. It is liquid at temperatures slightly higher than room temperature (m.p. 34°C) and can be distilled without decomposition both at atmospheric pressure (b.p. 217°C/710 torr.) (b.p. 217°C/0.95 bar) and under vacuum.

It can therefore be entirely recycled at the end of the oligomerization reaction, after decomposition of the acid-alkenes complex which forms in the polymerization stage (e.g. simply by treatment with water).

The use of CF₃SO₃H H₂O in the oligomerization of olefins, with respect to the prior-art methods involving use of BF₃ complexes, has the following additional advantages :
- it does not create any corrosion problem;
- it does not require the use of strictly anhydrous monomer feeds, as CF₃SO₃H H₂O is stable in the presence of water;
- it allows a better dosing as, owing to its low melting point, it can be charged into the reactor as a liquid and, owing to its stability, it does not require particular precautions;
- it allows a faster decantation of the catalytic complex, which is a liquid, from the obtained oligomers.

In accordance therewith, the present invention relates to a process for the oligomerization of olefins characterised in that a straight internal olefin having 10 to 20 carbon atoms or a, mixture of said olefins is contacted at a temperature of from 30 to 80°C with a catalyst consisting of trifluoromethanesulphonic acid monohydrate.

The olefins which can suitably be employed in the oligomerization process of the present invention include straight or branched mono-olefinic hydrocarbons of general formula CₙH₂ₙ, containing an olefinically unsaturated bond in any position of the molecule.

According to the of the present invention, in view of the end use of the obtained products, the starting olefins or olefin mixtures will consist or substantially consist of straight internal olefins with from 10 to 20 carbon atoms.

Said internal olefins are generally commercially available as mixtures thereof, as an example as C ₁₁-C₁₄, C₁₁-C₁₂, or C₁₅-C₁₈ mixtures.

According to a preferred embodiment, the reaction is carried out in the absence of a solvent.

It is however possible, if desired, to carry out the oligomerization in the presence of suitable inert solvents.

In this case, solvents which might well be employed are, for instance, optionally halogenated, saturated, straight or branched, aliphatic or cycloaliphatic hydrocarbons, e.g. n-pentane, n-hexane, n-heptane, dichloroethane, carbon tetrachloride, etc., alkyl or cyclic ethers, e.g. dioxane, and tetrahydrofuran, and mixtures thereof.

The reaction is carried out under stirring, at a temperature of from 20 to 180°C, but preferably from 30 to 80 C and, more preferably, from 40 to 60°C.

The amount of catalyst to be employed is typically from 2 to 30 %, and preferably from 10 to 20 %, by volume.

Amounts of catalyst higher than 30 % might, in principle, be employed as they do not create any problem as far as recovery of the products or recycle of the catalyst are concerned, but they increase the cost of the process without producing any additional improvement.

The reaction time may vary from 1 to 10 hours, depending on the reaction temperature and the amount of catalyst employed.

At the end of the reaction, the mixture is allowed to decantate, optionally cooled, and two phases, the oligomers and the catalyst, split out.

The catalyst is then separated by treatment with water and the unreacted monomers are removed from the oligomers, mainly dimers and trimers, by under vacuum distillation, typically by removing the fraction which distillates at a temperature up to 190°C/0.1 torr (190°C/1.3·10⁻⁴ bar).

The distillation residue, consisting of the oligomers, may be analysed and tested as to its rheological properties or it can be distilled under vacuum at temperatures of from 200 to 240°C.

The thus obtained oligomers still contain olefinic unsaturations. For their use in the synthetic lubricant field, these double bonds must be hydrogenated as known in the art (see for instance Italian patent application 25811 A/77).

The rheological characteristics of the oligomers are substantially unaltered by the hydrogenation step. The working examples which follow better illustrate some representative embodiments of the present invention but they are not intended to represent a limitation to the scopes thereof.

All the examples are carried out starting from the same mixture of internal C₁₁-C₁₂ olefins, previously distilled to remove any possible oxidation side-products.

The composition of the mixture, as determined by gas-chromatography, is : C₁₁ = 44 % and C₁₂ = 56 % by weight.

Trifluoromethanesulphonic acid monohydrate is prepared by adding 10.7 % of water to the anhydrous acid and is used immediately, before the heat of hydration is dispersed and the mass cools off and solidifies (m.p. 34°C).

Gas-chromatographic analyses of the obtained oligomers are carried out under the following conditions : fused silica capillary column (30 m x 0.3 mm id) packed with silicone OV 101; column starting temperature 60°C; column end temperature 300°C; temperature increase 3°C/min.

As for the carrier, hydrogen is used with an optimum linear velocity of 35 cm/sec.

Quantitative analysis of the oligomers is carried out with the internal standard method using n-C₁₃ and n-C₂₄.The number of olefinic unsaturations is expressed as Bromine Number (representing the amount of free bromine, in grams, which is consumed in reaction with 100 grams of the sample) and is determined as described in Organic Analysis, Vol.III, Intersc. Publ., New York, 1956).

The number of olefinic unsaturations is determined both on the starting monomers (Bromine Number = 95) and on the obtained oligomers.

### Example 1

A mixture of internal C₁₁-C₁₂ olefins (C₁₁ = 44 % and C₁₂ = 56 % by weight) (78 g corresponding to 100 ml), is charged into a 500-ml glass reactor, thermostated with a water bath, and equipped with a mechanical stirrer, a thermometer and a dripping funnel. The mixture is heated under stirring (700 rpm) to 40°C and CF₃SO₃H H₂O (19.5 g, 20 % by volume with respect to the volume of the overall reaction mixture) is added thereto by dripping it from the dripping funnel within 10 minutes. After eight hours the reaction is stopped by blocking the mechanical stirrer, removing the water bath and cooling the reaction mixture.

The reaction mixture is then allowed to decantate until two phases which can be easily distinguished by the different colors, dark red for the catalytic complex and light red for the oligomer phase, separate out. Separation of the two phases is achieved by means of a separatory funnel.

The oligomer phase is then washed twice with aqueous 2 % sodium hydroxide and once with water, affording a mixture (69.2 g) with the following composition (as determined by GC) : starting monomers = 12.07 %, dimers = 48.41 %, trimers = 28.6 %, tetramers = 4.35 %, higher oligomers = 6.57 %;
Bromine Number = 20.2.

The dark red phase (23 g) containing the catalytic complex is washed with water and the organic material contained therein is recovered (6.1 g) and analysed. Its % composition is as follows : unreacted monomers = 6.44, dimers = 29.0, trimers = 33.77, tetramers = 2.61, higher oligomers = 28.19;
Bromine Number = 65.

The overall yield in oligomers, with respect to the starting monomers, is 85.3 %.

### Example 2

By following substantially the same procedure as in Example 1 but heating to 50°C instead of 40°C, a mixture (68.1 g) with the following % composition is obtained : unreacted monomers = 9.14, dimers = 38.84, trimers = 32.8, tetramers = 5.51, higher oligomers = 14.7.
Bromine Number = 20.6.

The organic oil, separated from the catalytic complex as described in Example 1, (5.5 g) consists of : unreacted monomers = 3.02 %, dimers = 27.57 %, trimers = 29.61 %, tetramers = 2.64 %, higher oligomers = 37.34 %;
Bromine Number = 91.

The overall yield in oligomers, with respect to the starting monomers, is 87 %.

### Example 3

The procedure of Examples 1 and 2 is substantially repeated but heating to 60 C.

An oligomer mixture (70.2 g) with the following % composition is obtained : unreacted monomers = 9.9, dimers = 31.47, trimers = 35.5, tetramers = 7.61, higher oligomers = 15.52;
Bromine Number = 16.8.

The organic oil, separated from the catalytic complex by decomposition with water, (5.7 g) consists of : unreacted monomers = 2.78 %, dimers = 26.42 %, trimers = 28.04 %, tetramers = 3.07 %, higher oligomers = 39.69 %;
Bromine Number = 99.

The overall yield in oligomers, with respect to the starting monomers, is 88.2 %.

### Example 4

By following substantially the same procedure of the foregoing example but using 8.65 g of trifluoromethanesulphonic acid monohydrate (= 10 % with respect to the charge) instead of 19.5 g, an oligomer mixture (70.5 g) with the following % composition is obtained : unreacted monomers = 35.20, dimers = 50.56, trimers = 13.78, tetramers = 0.46, higher oligomers = none;
Bromine Number = 45.

The organic oil, separated from the catalytic complex by decomposition with water, (4.6 g) has the following % composition : unreacted monomers = 2.78, dimers = 26.42, trimers = 28.04, tetramers = 3.07, higher oligomers = 39.69.
Bromine Number = 99.

The overall yield in oligomers, with respect to the starting monomers, is 64.3 %.

## Claims

1. A process for the oligomerization of olefins characterised in that a straight internal olefin having 10 to 20 carbon atoms or a mixture of said olefins is contacted at a temperature of from 30 to 80°C with a catalyst consisting of trifluoromethanesulphonic acid monohydrate.

2. The process of claims 1 characterised in that the reaction is carried out in the absence of solvents.

3. The process of claim 1 characterised in that the reaction is carried out in the presence of a solvent selected from optionally halogenated, saturated, aliphatic and cycloaliphatic hydrocarbons, alkyl or saturated cyclic ethers, and mixture thereof.

4. The process of any of the preceding claims characterised in that trifluoromethanesulphonic acid monohydrate is employed in an amount of from 2 to 30% by volume with respect to the overall volume of the reaction mixture.

5. The process of claim 4 characterised in that the catalyst is employed in an amount of from 10 to 20% by volume with respect to the overall volume of the reaction mixture.

6. The process of any of the preceding claims further characterised in that the thus obtained oligomeric mixture undergoes hydrogenation.

## Patentansprüche

1. Verfahren zur Oligomerisierung von Olefinen, dadurch gekennzeichnet, daß ein geradkettiges inneres Olefin mit 10 bis 20 Kohlenstoffatomen oder ein Gemisch solcher Olefine bei einer Temperatur von 30 bis 80°C mit einem Katalysator zusammengebracht wird, bestehend aus Trifluormethansulfonsäure-monohydrat.

2. Verfahren nach Anspruch 1, dadurch gekennzeichnet, daß die Reaktion in Abwesenheit von Lösungsmitteln durchgeführt wird.

3. Verfahren nach Anspruch 1, dadurch gekennzeichnet, daß die Reaktion in Gegenwart eines Lösungsmittels durchgeführt wird, ausgewählt aus gegebenenfalls halogenierten, gesättigten, aliphatischen und cycloaliphatischen Kohlenwasserstoffen, Alkyl- oder gesättigten cyclischen Ethern und Gemischen davon.

4. Verfahren nach einem der vorangehenden Ansprüche, dadurch gekennzeichnet, daß das Trifluormethansulfonsäure-monohydrat in einer Menge von 2 bis 30 Vol.-%, bezogen auf das Gesamtvolumen des Reaktionsgemisches, angewandt wird.

5. Verfahren nach Anspruch 4, dadurch gekennzeichnet, daß der Katalysator in einer Menge von 10 bis 20 Vol.-%, bezogen auf das Gesamtvolumen des Reaktionsgemisches, angewandt wird.

6. Verfahren nach einem der vorangehenden Ansprüche, ferner dadurch gekennzeichnet, daß das so erhaltene oligomere Gemisch der Hydrierung unterworfen wird.

## Revendications

1. Procédé d'oligomérisation d'oléfines, **caractérisé** en ce que l'on met en contact une oléfine interne à chaîne droite, comportant de 10 à 20 atomes de carbone, ou un mélange de telles oléfines, avec un catalyseur constitué d'acide trifluorométhane-sulfonique monohydraté, à une température de 30 à 80°C.

2. Procédé selon la revendication 1, **caractérisé** en ce que la réaction est effectuée en l'absence de tout solvant.

3. Procédé selon la revendication 1, **caractérisé** en ce que la réaction est effectuée en présence d'un solvant choisi parmi les hydrocarbures aliphatiques ou cycloaliphatiques, saturés, éventuellement halogénés, les éthers cycliques saturés ou les éthers d'alkyle, et les mélanges de ces solvants.

4. Procédé selon l'une quelconque des revendications précédentes, **caractérisé** en ce que l'on utilise l'acide trifluorométhane-sulfonique monohydraté en une quantité représentant de 2 à 30 % en volume, par rapport au volume global du mélange réactionnel.

5. Procédé selon la revendication 4, **caractérisé** en ce que l'on utilise le catalyseur en une quantité représentant de 10 à 20 % en volume, par rapport au volume global du mélange réactionnel.

6. Procédé selon l'une quelconque des revendications précédentes, **caractérisé** en outre en ce que le mélange d'oligomères ainsi obtenu subit une hydrogénation.
